Europäisches Patentamt

(19) European Patent Office

Office européen des brevets

(11) Veröffentlichungsnummer : **0 082 798 B2**

(12) **NEUE EUROPÄISCHE PATENTSCHRIFT**

(45) Veröffentlichungstag der neuen Patentschrift :
27.11.91 Patentblatt 91/48

(51) Int. Cl.⁵ : **A61L 2/18, G02C 13/00, A01N 59/00**

(21) Anmeldenummer : 82710055.3

(22) Anmeldetag : 08.12.82

(54) **Verfahren zur Desinfektion und Reinigung von Kontaktlinsen.**

Die Akte enthält technische Angaben, die nach dem Eingang der Anmeldung eingereicht wurden und die nicht in dieser Patentschrift enthalten sind.

(30) Priorität : 21.12.81 DE 3150638

(43) Veröffentlichungstag der Anmeldung :
29.06.83 Patentblatt 83/26

(45) Bekanntmachung des Hinweises auf die Patenterteilung :
04.11.87 Patentblatt 87/45

(45) Bekanntmachung des Hinweises auf die Entscheidung über den Einspruch :
27.11.91 Patentblatt 91/48

(84) Benannte Vertragsstaaten :
AT BE CH DE FR GB IT LI NL SE

(56) Entgegenhaltungen :
DE-A- 2 417 844
DE-A- 2 425 714
DE-A- 2 810 978
FR-A- 2 231 395
GB-A- 1 531 035
US-A- 3 282 702
US-A- 3 700 761
US-A- 4 096 870
MERCK INDEX, 9. Edition, 1976, Seiten 242,243, Merck & Co., Rahway, USA
CHEMICAL ABSTRACTS, Band 83, Nr. 13, 29. September 1975, Seite 401, Nr. 112398f, Columbus, Ohio, USA
CHEMICAL ABSTRACTS, Band 87, Nr. 11, 12. September 1977, Seite 334, Nr. 81798n, Columbus, Ohio, USA,K.C. BHUYAN et al.: "The relative functions of superoxide dismutase and catalase in the eye"
D. Scott: Oxidoreductases in G. Reed (Herausgeber) Enzyme in Food Processing, 2. Aufl., Seiten 222-254; Academie Press, New York, 1975
Dr. Arnold Heins und Dr. Dieter Kühling "Aktivatoren und Katalysatoren für die Oxidation mit Wasserstoffperoxid (Kurzfassung eines Vortrags vor dem Ortsverband Marl-Hüls der Gesellschaft Deutscher Chemiker am 3. Februar 1971).

(56) Entgegenhaltungen :
Hans v. Euler "Chemie der Enzyme" zweiter Teil/3. Abschnitt, die Katalasen und die Enzyme der Oxydation und Reduktion (München, Verlag von J.F. Bergmann 1934), Seiten 1 bis 9, 30, 31.
W.L. Levine et al, "Disinfection of Hydrophilic Contact Lenses with Commercial Preparations of 3% and 6% Hydrogen Peroxide", Developments in Industrial Microbiology, Volume 22, 1981,Seiten 813 bis 818.
Seymour S. Block, "Disinfection, Sterilization and Preservation" 2. Auflage 1977, Lea und Febinger, Philadelphia, Seiten 677 bis 681.
Bitonte "Symposium on the Flexible Lens", Artikel "Solutions for Contact Lenses (Rigid and Flexible), Seite 210, Mosby 1972.
AFNOR (Recueil de normes francaises des antiseptiques et désinfectants), 1981, page 14
APPLIED MICROBIOLOGY, Oct. 1973, Sporicidal Properties of Hydrogen Peroxide Against Food Spoilage Organisms, R.T. Toledo et al., page 593
J. DAIRY RES. (1968), 35, 423: "The sterilizing effect against Bacillus subtilis spores of hydrogen peroxide at different temperatures and concentrations"
J. Starka "Physiologie und Biochemie der Mikrooganismen" (1968), Seiten 201-204
G. Eichhorn, "Inorganic Biochemistry", Band 2 (1973), Seiten 1015-1019
Reed, "Enzymes in Food Processing", 2.Auflage (1975), Seiten 247-254

(73) Patentinhaber : CIBA-GEIGY AG
CH-4002 Basel (CH)

(72) Erfinder : Giefer, Günter
Am Hirschgraben 30
W-6056 Heusenstamm 2 (DE)

(74) Vertreter : Foraita, Hans-Günther et al
Patentabteilung CIBA-GEIGY AG
CH-4002 Basel (CH)

**Beschreibung**

Die Erfindung betrifft ein Verfahren zur Desinfektion und Reinigung von Kontaktlinsen für das menschliche Auge, bei welchem Verfahren zunächst während eines ersten Zeitabschnittes eine Wasserstoffperoxid-Lösung auf die Kontaktlinse zur Einwirkung gebracht wird und anschliessend während eines zweiten Zeitabschnittes das an der Kontaktlinse haftende und/oder das in dieser aufgenommene Wasserstoffperoxid unter Wirkung eines Katalysators in Wasser und Sauerstoff aufgespalten wird, sowie die Verwendung einer Behandlungsflüssigkeit zur Endreinigung von Kontaktlinsen, die in einer Wasserstoffperoxid-Lösung eine Hygienebehandlung unterworfen wurden.

Beim Gebrauch von Kontaktlinsen entsteht eine Verunreinigung der Linsen, bei der die Ansammlung pathogener Keime eine kritische Grenze nicht überschreiten darf, um die Gefahr einer Infektion des Auges zu vermeiden. Aus diesem Grunde ist eine tägliche Desinfektion von Kontaktlinsen erforderlich. Dies gilt insbesondere für die heute weit verbreiteten hydrophilen Weichlinsen.

Die FR-AS-2 231 395 beschreibt zu diesem Zweck ein Verfahren, bei welchem Wasserstoffperoxid verwendet wird, welches gleichzeitig eine oxidierende, keimtötende, reinigende und geruchsbeseitigende Wirkung hat. Bei diesem Verfahren werden die Kontaktlinsen zunächst der Einwirkung einer 3 bis 30-prozentigen Lösung aus Wasserstoffperoxid ausgesetzt, was, wie in der GB-AS-1 531 035 beschrieben, beispielsweise in einem kleinen Behälter geschehen kann, in welchem die Kontaktlinsen in einem Tragkorb angeordnet sind. Die Zeitspanne, während der das Wasserstoffperoxid auf die Kontaktlinsen zur Erzielung einer ausreichenden Desinfektion und Reinigung einwirken muß, beträgt etwa 20 Minuten. Anschließend muß das an den Kontaktlinsen haftende Wasserstoffperoxid entfernt werden, was bei dem bekannten Verfahren dadurch geschieht, daß die Kontaktlinsen in einen zweiten Behälter gebracht werden, in welchem sich eine neutrale Flüssigkeit und ein Katalysator befinden, bei dessen Anwesenheit das Wasserstoffperoxid gemäss der Reaktionsgleichung.

$$2 H_2O_2 \rightarrow 2 H_2O + O_2$$

in Wasser und Sauerstoff aufgespalten wird. Bei dem Katalysator handelt es sich um einen metallbeschichteten festen Körper.

Der Nachteil des bekannten Verfahrens besteht darin, daß der zweite Verfahrensschritt, also die Beseitigung des Wasserstoffperoxids durch Aufspaltung, relativ lange dauert. Es sind hierfür mindestens 4 Stunden erforderlich. Vergißt der Benutzer von Kontaktlinsen beispielsweise am Abend zuvor, die Kontaktlinsen aus der Wasserstoffperoxid-Lösung in die den Katalysator enthaltende Lösung umzuplazieren, so fehlt am folgenden Morgen die Zeit, die Wasserstoffperoxid-Reste von den Kontaktlinsen in solchem Maße zu entfernen, daß ein Brennen der Augen beim Tragen der Kontaktlinsen vermieden wird. Gemäß einer Abwandlung des bekannten Verfahrens, die ebenfalls der FR-AS-2 231 395 zu entnehmen ist, wird der metallische Katalysator der Wasserstoffperoxid-Lösung bereits in dem Augenblick zugegeben, in welchem die Wasserstoffperoxid-Lösung zur Einwirkung auf die Kontaktlinsen gebracht wird. Der Desinfektions- und Reinigungsvorgang der Kontaktlinsen und die Zersetzung des Wasserstoffperoxids durch den Katalysator beginnen also gleichzeitig. Dies ist bei diesem bekannten Verfahren möglich, da der vom Katalysator ausgelöste Zersetzungsvorgang langsam abläuft. Auch dieses einstufige Verfahren hat den wesentlichen Nachteil, dass es sehr lange (mehrere Stunden) dauert. Darüberhinaus ist bei diesem bekannten Verfahren zur Beseitigung verbliebener Reste von Wasserstoffperoxid abschliessend noch eine Spülung in Salzlösung vorgesehen.

Aus dem Artikel "Disinfection of Hydrophilic Contact Lenses with Commercial Preparations of 3 % and 6 % Hydrogen Peroxide" von W.L. Levine et al., Dev. Ind. Microbiol. 22, 613 (1981) ist bekannt, dass Kontaktlinsen mit Wasserstoffperoxid desinfiziert werden können. Bei dieser Untersuchung werden kontaminierte Linsen nach Desinfektion mit Wasserstoffperoxid in ein Kulturmedium eingebracht, um anhand des anschliessenden Bakterienwachstums festzustellen, ob und ggf. wieviele Bakterien durch die Wasserstoffperoxid-Behandlung nicht abgetötet worden sind. Weil die Anwesenheit von Wasserstoffperoxid in dem Kulturmedium das Bakterienwachstum behindert und daher falsche Ergebnisse bezüglich der Abtötungsraten des Wasserstoffperoxids bei dem Desinfektionsvorgang ergeben würde, wird dem Kulturmedium eine kleine Menge Katalase zugegeben, um das mitgeschleppte Peroxid zu zersetzen. Katalase wird hierbei als Bestandteil eines Kulturmediums erwähnt und dient dazu, das Wachstum von Bakterien zu ermöglichen.

Der Erfindung liegt die Aufgabe zugrunde, das eingangs genannte Verfahren derart weiterzuentwickeln, dass die erforderliche Zeitspanne für den zweiten Verfahrensschritt wesentlich verkürzt wird und zugleich eine sehr hochgradige Entfernung von Wasserstoffperoxid erzielt wird.

Zur Lösung dieser Aufgabe wird erfindungsgemäss ein Verfahren vorgeschlagen, welches dadurch gekennzeichnet ist, dass nach Abschluss des ersten Zeitabschnittes die die Kontaktlinse umgebende Wasserstoffperoxid-Lösung durch eine der Konzentration der Augenflüssigkeit entsprechende Kochsalzlösung ersetzt wird, welche Katalase enthält.

Gemäss einer vorteilhaften Weiterbildung der Erfindung wird als Kochsalzlösung, welche Katalase enthält, eine Mischung von zumindest 100 bis 1000, vorzugsweise 400 bis 600 Volumenteilen einer etwa 0,8 bis 1,0 %-igen Kochsalzlösung und einem Volumenteil einer handelsüblichen hochkonzentrierten Katalase-Lösung verwendet. Die Ansprüche 3 und 4 sind auf die Verwendung einer Behandlungsflüssigkeit zur Durchführung des erfindungsgemässen Verfahrens gerichtet.

Katalase ist ein Enzym, welches vorzugsweise aus Rinderleber gewonnen wird und eine kristalline Struktur hat.

Sie ist im Handel als hochkonzentrierte Lösung in 30-prozentigem Glycerin und 10-prozentigem Äthanol gelöst erhältlich und findet in der Lebensmittelindustrie Verwendung, insbesondere bei der Milchbehandlung, wie dies z. B. in der US-A-3 282 702 beschrieben ist.

Bei der Verwendung der organischen Katalase besteht an sich das Problem, daß diese nicht sehr beständig ist und ihre Aufbewahrung schwierig ist. Die im Handel erhältliche, oben genannte hochkonzentrierte Katalase-Lösung muß bei 4° C gelagert werden und ist auch dann nur mehrere Monate haltbar.

Die Erfindung beruht auf der Erkenntnis, daß die Beseitigung der Wasserstoffperoxidreste an den Kontaktlinsen in Gegenwart von Katalase als Katalysator wesentlich schnellen und gründlicher stattfindet, als bei Verwendung der bekannten Katalysatoren, daß bei der Verwendung der Katalase in der isotonen Kochsalzlösung die Katalase sich überraschenderweise als sehr lange haltbar und wenig temperaturanfällig erweist sowie daß die Linsen unmittelbar im Anschluß an die Behandlung ohne weitere Spülung ins Auge eingesetzt werden können. All dies macht das Verfahren nach der Erfindung für seine praktische Anwendbarkeit außerordentlich geeignet.

Bei dem Verfahren nach der Erfindung beträgt die Zeitspanne für den zweiten Verfahrensabschnitt nur wenige Minuten (etwa 5 Minuten). Bei dem Verfahren gemäß der Erfindung spielt es somit keine Rolle, wenn der Benutzer von Kontaktlinsen abends vergißt, den zweiten Verfahrensschritt einzuleiten, da für seine Nachholung am folgenden Morgen nur wenige Minuten erforderlich sind. Durch die sehr stürmische Zersetzung des Wasserstoffperoxids wird aber nicht nur die erforderliche Zeit wesentlich verkürzt; es kommt auch an der Oberfläche der Kontaktlinse zu einer explosionsartigen Zersetzung des Wasserstoffperoxids, welche die während des ersten Verfahrensschrittes brüchig gewordenen Verunreinigungen an der Oberfläche der Kontaktlinsen absprengt. Die Beseitigung der Wasserstoffperoxidreste bei dem Verfahren nach der Erfindung ist so gründlich, daß auf eine anschließende Nachbehandlung (Spülung) der Linsen in einer Salzlösung verzichtet werden kann.

Bei der Durchführung des zweiten Verfahrensschrittes, d.h. der katalytischen Zersetzung des Wasserstoffperoxids, verwendet man am besten als Flüssigkeit, welche die Wasserstoffperoxid-Lösung in dem Behälter ersetzt, die oben genannte, aus in Kochsalzlösung aufbewahrter hochkonzentrierter Katalase bestehende Flüssigkeit. Es ist auch möglich, mit zwei Behältern zu arbeiten, wobei in einem Behälter die Wasserstoffperoxid-Lösung und in dem anderen Behälter die Katalase enthaltende Kochsalzlösung ist, deren Konzentration der Konzentration der Augenflüssigkeit entspricht, d.h. die isoton ist.

Die Konzentration, in welcher die Katalase in ihrer handelsüblichen Form der Trägerflüssigkeit, z. B. Wasserstoffperoxid-Lösung oder Kochsalzlösung, zugesetzt wird, kann in weiten Grenzen von etwa 0, 1 bis 1 -Volumenprozent schwanken.

**Patentansprüche**

1. Verfahren zur Desinfektion und Reinigung von Kontaktlinsen für das menschliche Auge, bei welchem Verfahen zunächst während eines ersten Zeitabschnitts eine Wasserstoffperoxid-Lösung auf die Kontaktlinse zur Einwirkung gebracht wird und anschliessend während eines zweiten Zeitabschnittes das an der Kontaktlinse haftende und/oder das in dieser aufgenommene Wasserstoffperoxid unter Wirkung eines Katalysators in Wasser und Sauerstoff aufgespalten wird, dadurch gekennzeichnet, dass nach Abschluss des ersten Zeitabschnittes die die Kontaktlinse umgebende Wasserstoffperoxid-Lösung durch eine der Konzentration der Augenflüssigkeit entsprechende Kochsalzlösung ersetzt wird, welche Katalase enthält.

2. Verfahren nach Anspruch 1, dadurch gekennzeichnet, dass als Kochsalzlösung, welche Katalase enthält, eine Mischung von zumindest 100 bis 1000 Volumenteilen einer etwa 0,8 bis 1,0 %-igen Kochsalzlösung und einem Volumenteil einer handelsüblichen hochkonzentrierten Katalase-Lösung verwendet wird.

3. Verwendung einer Behandlungsflüssigkeit zur Endreinigung von Kontaktlinsen, die in einer Wasserstoffperoxid-Lösung einer Hygienebehandlung unterworfen wurden, welche Flüssigkeit in einer der Konzentration der Augenflüssigkeit entsprechenden Kochsalzlösung Katalase enthält, zur Aufspaltung von an der Kontaktlinse haftendem und/oder in dieser aufgenommenen Wasserstoffperoxid in Wasser und Sauerstoff.

4. Verwendung gemäss Anspruch 3, dadurch gekennzeichnet, dass die Behandlungsflüssigkeit aus einer Mischung von zumindest 100 bis 1000 Volumenteilen einer etwa 0,8 bis 1,0 %-igen Kochsalzlösung und einem Volumenteil einer handelsüblichen hochkonzentrierten Katalase-Lösung besteht.

## Claims

1. Method for the disinfection and cleaning of contact lenses for the human eye, in which method during an initial period a hydrogen peroxide solution is caused to act on the contact lens and then, during a second period, the hydrogen peroxide adhering to and/or absorbed by the contact lens is split under the action of a catalyst into water and oxygen, characterised in that after the end of the initial period the hydrogen peroxide solution surrounding the contact lens is replaced by a solution of sodium chloride corresponding to the concentration of the eye fluid and containing catalase.

2. Method according to claim 1, characterised in that as the sodium chloride solution containing catalase a mixture of at least from 100 to 1000 parts by volume of a solution containing about 0.8 to 1.0 % of sodium chloride and 1 part by volume of a commercially available highly concentrated solution of catalase is used.

3. Use of a treatment liquid for the final cleaning of contact lenses which have been subjected to a hygiene treatment in a hydrogen peroxide solution, which liquid comprises catalase in a solution of sodium chloride corresponding to the concentration of the eye fluid, for the decomposition of the hydrogen peroxide adhering to and/or absorbed by the contact lens into water and oxygen.

4. Use according to claim 3, characterised in that the treatment liquid consists of a mixture of at least from 100 to 1000 parts by volume of a solution containing about 0.8 to 1.0 % of sodium chloride and 1 part by volume of a commercially available highly concentrated catalase solution.

## Revendications

1. Procédé pour désinfecter et nettoyer des lentilles de contact pour l'oeil humain dans lequel on fait d'abord agir, dans un premier temps, une solution de peroxyde d'hydrogène sur la lentille de contact puis, dans un deuxième temps, on décompose le peroxyde d'hydrogène adhérant à la lentille de contact et/ou absorbé dans cette lentille en eau et oxygène sous l'action d'un catalyseur, caractérisé en ce que, après la première opération, on remplace la solution de peroxyde d'hydrogène qui imprègne la lentille de contact par une solution de chlorure de sodium à une concentration correspondant à celle du liquide oculaire et qui contient de la catalase.

2. Procédé selon la revendication 1, caractérisé en ce que l'on utilise en tant que solution de chlorure de sodium contenant de la catalase un mélange d'au moins 100 à 1000 parties en volume d'une solution de chlorure de sodium à une concentration d'environ 0,8 à 1,0 % et d'1 partie en volume d'une solution de catalase à haute concentration du commerce.

3. Utilisation du liquide de traitement pour le nettoyage final des lentilles de contact qui ont été soumises à un traitement hygiénique dans une solution de peroxyde d'hydrogène, caractérisé en ce qu'il contient de la catalase dans une solution de chlorure de sodium à une concentration correspondant à celle du liquide oculaire pour la décomposition du peroxyde d'hydrogène adhérant à la lentille de contact et/ou absorbé dans cette lentille en eau et oxygène.

4. Utilisation selon la revendication 3, caractérisé en ce que le liquide de traitement consiste en un mélange d'au moins 100 à 1000 parties en volume d'une solution de chlorure de sodium à une concentration d'environ 0,8 à 1,0 % et d'1 partie en volume d'une solution de catalase à haute concentration du commerce.